# EUROPÄISCHE PATENTSCHRIFT

(11) **EP 0 281 902 B1**
(45) Veröffentlichungstag und Bekanntmachung des Hinweises auf die Patenterteilung: **09.06.1993**
(21) Anmeldenummer: 88103097.7
(22) Anmeldetag: 02.03.1988
(51) Int. Cl.: C07D 221/14, A61K 31/435

(54) **Bis-naphthalimide, ihre Herstellung und Verwendung**
Bis-naphthalimides, their preparation and use
Bis-naphtalimides, leur préparation et leur utilisation

(30) Priorität: 10.03.1987 DE 3707651
(43) Veröffentlichungstag der Anmeldung: 14.09.1988
(73) Patentinhaber: KNOLL AG, D-67061 Ludwigshafen (DE)
(72) Erfinder: Fernandez Brana, Miguel, Dr., ES-28036 Madrid (ES); Castellano Berlanga, José Maria, Dr., ES-28036 Madrid (ES); Keilhauer, Gerhard, Dr., D-6701 Dannstadt-Schauernheim (DE); Schlick, Erich, Dr., D-6708 Neuhofen (DE)
(74) Vertreter: Karau, Wolfgang, Dr.

(56) Entgegenhaltungen:
- DE-A- 2 823 987
- US-A- 4 499 266
- ARZNEIMITTELFORSCHUNG/DRUG RESEARCH 34(II),1243 (1984)

## Beschreibung

Die Erfindung betrifft Bis-naphthalimide, ein Verfahren zu deren Herstellung sowie deren Verwendung bei der Bekämpfung von Krankheiten.

Es ist bereits bekannt, daß bestimmte Benzo[de]isochinoline tumorhemmende Eigenschaften besitzen (Arzneim.Forsch./Drug Research 34 (II), 1243 (1984). Die Wirkungen dieser Verbindungen sind jedoch nicht in jeder Hinsicht befriedigend.

Es wurde nun gefunden, daß Bis-naphthalimide der Formel I 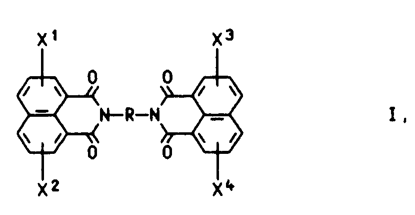
worin
- X¹, X², X³ und X⁴: gleich oder verschieden sind und Wasserstoff, Nitro, Amino, C₁-C₆-Alkylamino, Di-C₁-C₆-alkylamino, Hydroxy, C₁-C₆-Alkoxy, Halogen, Trihalogenmethyl, C₁-C₆-Alkyl, Formyl, C₁-C₆-Alkylcarbonyl, C₁-C₆-Acylamino, Ureyl, C₁-C₆-Alkylureyl, C₁-C₆-Alkoxycarbonylamino bedeuten und
- R: einen geraden oder verzweigten Alkylenrest bedeutet, der durch mindestens 1 Stickstoffatom unterbrochen ist, und worin 2 Stickstoffatome zusätzlich durch eine Alkylengruppe miteinander verbunden sein können,
sowie deren Salze mit physiologisch verträglichen Säuren, eine bessere Wirkung bzw. ein besseres Wirkungsspektrum als tumorhemmende Substanzen besitzen und antileukämisch wirken.

Bevorzugt sind die Verbindungen, in denen die Reste X¹ und X³ Amino-, Acetylamino- oder Nitrogruppen und die Reste X² und X⁴ Wasserstoffatome oder Amino-, Acetylamino- oder Nitrogruppen darstellen. Von diesen Verbindungen sind solche besonders hervorzuheben, in denen X¹ und X³ Amino- oder Acetylaminogruppen und X² und X³ Nitrogruppen sind.

Als physiologisch verträgliche Säuren eignen sich zur Salzbildung organische und anorganische Säuren, wie Salzsäure, Schwefelsäure, Phosphorsäure, Essigsäure, Zitronensäure, Oxalsäure, Malonsäure, Salizylsäure, Maleinsäure, Fumarsäure, Bernsteinsäure, Ascorbinsäure, Äpfelsäure, Methansulfonsäure, Isethionsäure, Milschsäure, Gluconsäure, Glucuronsäure, Amidosulfonsäure, Benzoesäure, Weinsäure, Pamoasäure.

Die neuen Verbindungen können in solvatisierter Form vorliegen. Solche Formen können sich beispielsweise mit Wasser oder Ethanol bilden.

Die neuen Verbindungen werden hergestellt, indem man ein 1,8-Naphthalsäureanhydrid der Formel II 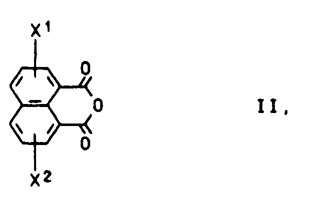
worin X¹ und X² die angegebene Bedeutung besitzen, und ein 1,8-Naphthalsäureanhydrid der Formel III 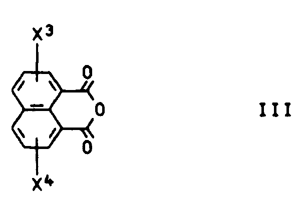
worin X³ und X⁴ die angegebene Bedeutung besitzen, mit einem Amin der Formel IV

H₂N-R-NH₂ IV,

worin R die angegebene Bedeutung besitzt, umsetzt und die so erhaltenen Verbindungen gegebenenfalls in deren Salze mit physiologisch verträglichen Säuren überführt.

Die Umsetzung wird in einem geeigneten Lösungsmittel wie Dimethylformamid, Methanol, Ethanol oder Propanol in der Regel bei Raumtemperatur durchgeführt. Die erfindungsgemäße Verbindung scheidet sich aus dem Reaktionsgemisch ab und kann chromatographisch und/oder durch Umkristallisation gereinigt werden.

Die so erhaltenen Verbindungen können in an sich bekannter Weise in ihre Salze überführt werden, z.B. durch Umsetzen mit einer Säure.

Die neuen Verbindungen besitzen eine gute cytotoxische Wirkung wie sich in folgendem Versuch zeigen läßt: Tumorzellen menschlicher Herkunft werden auf Mikrotiterplatten in einer Konzentration von 1 bis 5 x 10³ Zellen pro Vertiefung ausplattiert und im Brutschrank bei 37°C und unter einer mit Wasserdampf gesättigten Normalatmosphäre, jedoch unter Zusatz von 5 % Kohlendioxid, über Nacht in einem Vollmedium vorinkubiert. Die zu testenden Substanzen werden in demselben Vollmedium gelöst und aus den Lösungen Verdünnungsreihen (Verdünnungsverhältnis 1:2) hergestellt. Jeweils 0,1 ml der so erhaltenen Lösungen werden in die Vertiefungen der Mikrotiterplatten gegeben. Die Platten werden erneut für 72 h bei 37°C inkubiert. Danach werden adhärente Zellen mit Kristallviolett und nicht-adhärente Zellen mit MTT (Tetrazolium) angefärbt und deren Extinktion bei 540 nm gemessen. Die Abnahme der Extinktion gegenüber einem Blindwert (erhalten ohne Testsubstanz) ist ein Maß für Cytotoxizität der Verbindungen.

Die neuen Verbindungen eignen sich daher zur Behandlung von festen Tumoren wie auch von bestimmten Formen von Leukämie.

Die erfindungsgemäßen Verbindungen können in üblicher Weise oral oder parenteral verabfolgt werden. Sie können in den gebräuchlichen galenischen Applikationsformen fest oder flüssig angewendet werden, z.B. als Tabletten, Filmtabletten, Kapseln, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 90 Gew.%.

### Beispiel 1

Zu 9,8 g (0,04 Mol) 3-Nitro-1,8-naphthalsäureanhydrid in 100 ml Dimethylformamid (= DMF) wurde eine Lösung von 2,62 g (0,02 Mol) 3,3-Diamino-di-(n-propyl)-amin in 50 ml DMF getropft. Die Mischung wurde bei Raumtemperatur 3 h gerührt. Das ausgeschiedene Produkt wurde abfiltriert und aus DMF umkristallisiert. Man erhielt 3,3-Bis-(3-nitro-1,8-naphthalimido)-di-(n-propyl)-amin, Fp. 215-217°C. Ausbeute 65 %.

Analog Beispiel 1 wurden hergestellt:
Methyl-3,3-bis-(3-nitro-1,8-naphthalimido)-di-(n-propyl)-amin,
2.
   Fp. 207°C (DMF), Ausbeute 76 %.
3. 2-(3-Nitro-1,8-naphthalimido)-ethyl-[3-(3-nitro-1,8-naphthalimido)-n-propyl]-amin, Fp. 237°C (DMF), Ausbeute 72 %.
4. 2,2'-Bis-(3-nitro-1,8-naphthalimido)-diethylamin, Fp. 235-237°C, Ausbeute 68 %.
5. 2-(3-Amino-1,8-naphthalimido)-ethyl-[3-(3-amino-1,8-naphthalimido)-n-propyl]-amin, Fp. 280°C (DMF), Ausbeute 56 %.
6. 3,3'-Bis-(4-nitro-1,8-naphthalimido)-di-(n-propyl)-amin, Fp. 205°C (DMF), Ausbeute 51 %.
7. 1,4-bis-[3-(3-nitro-1,8-naphthalimido)-n-propyl]-piperazin, Fp. 246°C (DMF), Ausbeute 55 %.
8. 1,4-Bis-[3-(3-amino-1,8-naphthalimido)-n-propyl]-piperazin, Fp. 313°C (DMF), Ausbeute 48 %.
9. 3,3'-Bis-(3,6-dinitro-1,8-naphthalimido)-di-(n-propyl)-amin, Fp. 350°C (DMF), Ausbeute 42 %.
10. 2,2'-Bis-(3-amino-1,8-naphthalimido)-diethylamin, Fp. 272° (DMF-Wasser), Ausbeute 57 %.
11. N,N'-Bis-[3-(3-amino-1,8-naphthalimido)-n-propyl] 1,4-diaminobutan, Fp. 192° (Xylenol), Ausbeute 45 %.
12. N,N'-Bis-[3-(3-amino-1,8-naphthalimido)-n-propyl]-ethylen-diamin, Fp. 220° (DMF), Ausbeute 68 %.
13. 3,3'-Bis-[3-amino-1,8-naphthalimido)-di-(n-propyl)-amin, Fp. 265° (DMF), Ausbeute 42 %.
14. Methyl-2,2'-bis-(3-nitro-1,8-naphthalimido)-diethylamin, Fp. 261° (DMF), Ausbeute 56 %.
15. Methyl-[2-(3-nitro-1,8-naphthalimido)-ethyl]-[3-(3-nitro-1,8-naphthalimido)-n-propyl]-amin, Fp. 205° (DMF), Ausbeute 62 %.
16. Methyl-3,3'-Bis-(3-amino-1,8-naphthalimido)-di-(n-propyl)-amin, Fp. 201° (Xylenol), Ausbeute 48 %.

## Patentansprüche (Patentansprüche für folgende(n) Vertragsstaat(en): AT, BE, CH, DE, FR, GB, IT, LI, NL)

1. Bis-naphthalimide der Formel I 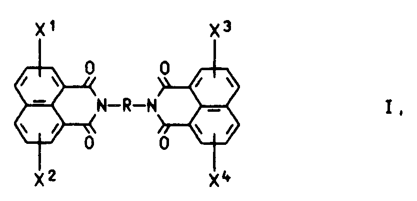 worin
X¹, X², X³ und X⁴ gleich oder verschieden sind und Wasserstoff, Nitro, Amino, C₁-C₆-Alkylamino, Di-C₁-C₆-alkylamino, Hydroxy, C₁-C₆-Alkoxy, Halogen, Trihalogenmethyl, C₁-C₆-Alkyl, Formyl, C₁-C₆-Alkylcarbonyl, C₁-C₆-Acylamino, Ureyl, C₁-C₆-Alkylureyl, C₁-C₆-Alkoxycarbonylamino ist und
R einen geraden oder verzweigten Alkylenrest bedeutet, der durch mindestens 1 Stickstoffatom unterbrochen ist, und worin 2 Stickstoffatome zusätzlich durch eine Alkylengruppe miteinander verbunden sein können,
sowie deren Salze mit physiologisch verträglichen Säuren.

2. Bis-naphthylamide der Formel I gemäß Anspruch 1 zur Verwendung bei der Bekämpfung von Krankheiten.

3. Verwendung der Bis-naphthylamide der Formel I gemäß Anspruch 1 zur Herstellung von Arzneimitteln, die das Tumorwachstum hemmen oder antileukämisch wirken.

## Patentansprüche (Patentansprüche für folgende(n) Vertragsstaat(en): ES)

1. Verfahren zur Herstellung von Bis-naphthalimiden der Formel I 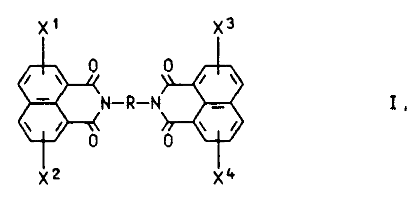 worin
X¹, X², X³ und X⁴ gleich oder verschieden sind und Wasserstoff, Nitro, Amino, C₁-C₆-Alkylamino, Di-C₁-C₆-alkylamino, Hydroxy, C₁-C₆-Alkoxy, Halogen, Trihalogenmethyl, C₁-C₆-Alkyl, Formyl, C₁-C₆-Alkylcarbonyl, C₁-C₆-Acylamino, Ureyl, C₁-C₆-Alkylureyl, C₁-C₆-Alkoxycarbonylamino ist und
R einen geraden oder verzweigten Alkylenrest bedeutet, der durch mindestens 1 Stickstoffatom unterbrochen ist, und worin 2 Stickstoffatome zusätzlich durch eine Alkylengruppe miteinander verbunden sein können,
sowie deren Salzen mit physiologisch verträglichen Säuren, dadurch gekennzeichnet, daß man ein 1,8-Naphthalsäureanhydrid der Formel II 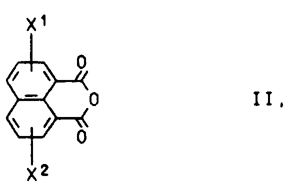 worin X¹ und X² die angegebene Bedeutung besitzen, und ein 1,8-Naphthalsäureanhydrid der Formel III 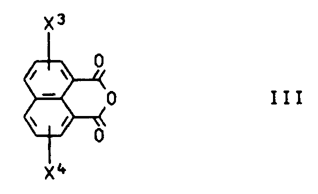 worin X³ und X⁴ die angegebene Bedeutung besitzen, mit einem Amin der Formel IV
H₂N-R-NH₂ IV,
worin R die angegebene Bedeutung besitzt, umsetzt und die so erhaltenen Verbindungen gegebenenfalls in deren Salze mit physiologisch verträglichen Säuren überführt.

## Claims (Claims for the following Contracting State(s): AT, BE, CH, DE, FR, GB, IT, LI, NL)

1. A bisnaphthalimide of the formula I 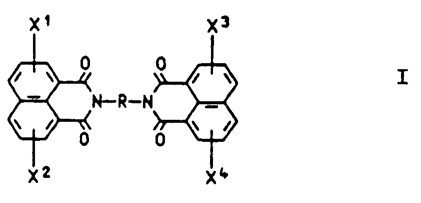 where
X¹, X², X³ and X⁴ are identical or different and are each hydrogen, nitro, amino, C₁-C₆-alkylamino, di-C₁-C₆-alkylamino, hydroxyl, C₁-C₆-alkoxy, halogen, trihalomethyl, C₁-C₆-alkyl, formyl, C₁-C₆-alkylcarbonyl, C₁-C₆-acylamino, ureyl, C₁-C₆-alkylureyl or C₁-C₆-alkoxycarbonylamino and
R is a straight-chain or branched alkylene radical which is interrupted by one or more nitrogen atoms, and where 2 nitrogen atoms may additionally be bonded to one another by an alkylene group,
and its salts with physiologically tolerated acids.

2. A bisnaphthalimide of the formula I as claimed in claim 1 for use in the treatment of disorders.

3. The use of a bisnaphthalimide of the formula I as claimed in claim 1 for the preparation of drugs which inhibit tumor growth or possess antileukemic activity.

## Claims (Claims for the following Contracting State(s): ES)

1. A process for the preparation of a bisnaphthalimide of the formula I 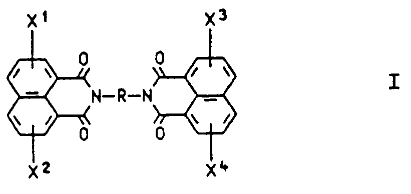 where
X¹, X², X³ and X⁴ are identical or different and are each hydrogen, nitro, amino, C₁-C₆-alkylamino, di-C₁-C₆-alkylamino, hydroxyl, C₁-C₆-alkoxy, halogen, trihalomethyl, C₁-C₆-alkyl, formyl, C₁-C₆-alkylcarbonyl, C₁-C₆-acylamino, ureyl, C₁-C₆-alkylureyl or C₁-C₆-alkoxycarbonylamino and
R is a straight-chain or branched alkylene radical which is interrupted by one or more nitrogen atoms, and where 2 nitrogen atoms may additionally be bonded to one another by an alkylene group,
and its salts with physiologically tolerated acids, which comprises reacting a 1,8-naphthalic anhydride of the formula II 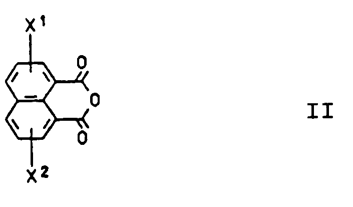 where X¹ and X² are each as defined above, and a 1,8-naphthalic anhydride of the formula III 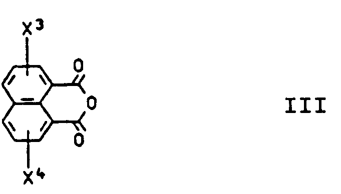 where X³ and X⁴ are each as defined above, with an amine of the formula IV
H₂N-R-NH₂ IV
where R is as defined above, and if desired converting the resulting compounds into their salts with physiologically tolerated acids.

## Revendications (Revendications pour l'(les) Etat(s) contractant(s) suivant(s): AT, BE, CH, DE, FR, GB, IT, LI, NL)

1. Bis-naphtalimides de formule I 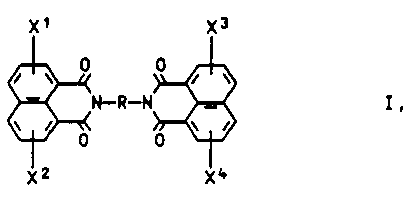 dans laquelle
X¹
X²
X³
X⁴ sont des groupements identiques ou différents et représentent hydrogène, nitro, amino, alkylamino en C1-C6, di-alkylamino en C1-C6, hydroxy, alkoxy en C1-C6, halogène, tri-halogénométhyle, alkyle en C1-C6, formyle, alkylcarbonyle en C1-C6, acylamino en C1-C6, uréyle, alkyluréyle en C1-C6, alkoxycarbonylamino en C1-C6 et
R représente un groupement alkylène droit ou ramifié qui est interrompu par au moins 1 atome d'azote,
et dans laquelle en outre 2 atomes d'azote peuvent être liés l'un à l'autre par un groupement alkylène,
de même que leurs sels avec des acides tolérés sur le plan physiologique.

2. Bis-naphtylamides de formule I selon la revendication 1 à employer dans la lutte contre les maladies.

3. Emploi des bis-naphtylamides de formule I selon la revendication 1 dans la fabrication de médicaments qui freinent la croissance des tumeurs ou qui agissent contre la leucémie.

## Revendications (Revendications pour l'(les) Etat(s) contractant(s) suivant(s): ES)

1. Procédé pour la fabrication des bis-naphtalimides de formule I 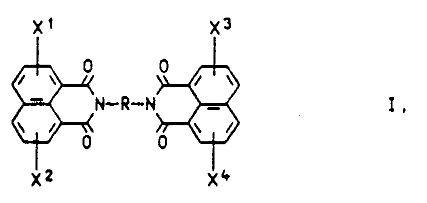 dans laquelle
X¹
X²
X³
X⁴ sont des groupements identiques ou différents et représentent hydrogène, nitro, amino, alkylamino en C1-C6, di-alkylamino en C1-C6, hydroxy, alkoxy en C1-C6, halogène, tri-halogénométhyle, alkyle en C1-C6, formyle, alkylcarbonyle en C1-C6, acylamino en C1-C6, uréyle, alkyluréyle en C1-C6, alkoxycarbonylamino en C1-C6 et
R représente un groupement alkylène droit ou ramifié qui est interrompu par au moins 1 atome d'azote,
et dans laquelle en outre 2 atomes d'azote peuvent être liés l'un à l'autre par un groupement alkylène,
de même que leurs sels avec des acides tolérés sur le plan physiologique, caractérisé en ce que l'on fait réagir un anhydride d'acide 1,8-naphtalique de formule II 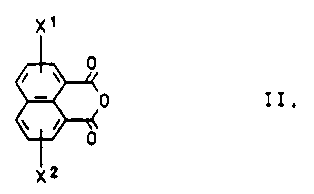 dans laquelle X¹ et X² possèdent la signification indiquée, et un anhydride d'acide 1,8-naphtalique de formule III 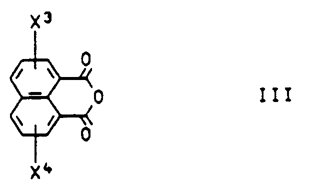 dans laquelle X³ et X⁴ possèdent la signification indiquée, avec une amine de formule IV
H₂N-R-NH₂ IV
dans laquelle R possède la signification indiquée, et en ce que l'on convertit les composés ainsi obtenus le cas échéant en sels de ces derniers avec des acides tolérés sur le plan physiologique.
